# EUROPÄISCHE PATENTSCHRIFT

(11) **EP 1 582 112 B1**
(45) Veröffentlichungstag und Bekanntmachung des Hinweises auf die Patenterteilung: **25.02.2009**
(21) Anmeldenummer: 05005818.9
(22) Anmeldetag: 17.03.2005
(51) Int. Cl.: A45D 26/00, A61N 1/34

(54) **Epilationsgerät mit schmerzhemmenden Mitteln und zugehöriges Verfahren**
Depilatory appliance comprising pain-relieving means and corresponding method
Dispositif d'épilation comportant des moyens antidouleur et procédé correspondant

(30) Priorität: 31.03.2004 DE 102004015762
(43) Veröffentlichungstag der Anmeldung: 05.10.2005
(73) Patentinhaber: Braun GmbH, 61476 Kronberg (DE)
(72) Erfinder: Juraschek, Reinhard, 61476 Kronberg (DE); Kahler, Elke, 37077 Göttingen (DE); Kampmeier, Jürgen, 91054 Buckenhof (DE)

(56) Entgegenhaltungen:
- DE-A1- 2 538 289
- DE-A1- 3 520 018
- DE-A1- 19 521 585
- DE-C1- 4 408 809

## Beschreibung

Die Erfindung bezieht sich auf ein Epilationsgerät und ein Epilationsverfahren, insbesondere zum Auszupfen von Haaren der menschlichen Haut.

Das Auszupfen von Haaren mittels eines Epilationsgeräts verursacht jeweils einen Schmerz, der abhängig vom subjektiven Schmerzempfinden des Benutzers sehr unangenehm sein kann und dazu führt, daß Epilationsgeräte von einigen Benutzern nur sehr ungern angewendet werden und potentielle Benutzer den Einsatz von Epilationsgeräten ganz ablehnen. Es wurde daher bereits versucht, durch Ausübung zusätzlicher Reize, insbesondere durch Anwenden eines elektrischen Stroms, den Epilationsvorgang für den Benutzer angenehmer zu gestalten. So ist aus der DE 44 08 809 C1 ein Epilationsgerät bekannt, das wenigstens zwei Elektroden aufweist, die während des Epilationsvorgangs mit der Haut in Kontakt bringbar sind und mit einem Generator zur Erzeugung eines Stimulierungsstroms verbunden sind. Der Generator ist insbesondere als ein einstellbarer Impulsgenerator ausgebildet, der unipolare Pulse bzw. einen pulsierenden Gleichstrom einer einstellbaren Intensität im Bereich von etwa 0 bis 200 mAs erzeugt. Die Pulsfolgefrequenz kann in einem Bereich zwischen etwa 1 Hz und 500 Hz, die Pulsdauer zwischen etwa 1 ms und 0,5 s und die Pulsamplitude zwischen etwa 1 V und 100 V variiert werden.

Aus der WO 97/00032 A1 ist es bekannt, bei einem Epilationsgerät ein Element vorzusehen, das einen mechanischen Impuls und/oder einen elektrischen Impuls abgibt. Der Impuls wird zeitlich oder örtlich bzw. zeitlich und örtlich unmittelbar vor dem jeweiligen individuellen Auszupfvorgang an bzw. auf die Haut abgegeben.

Die EP 0 986 319 B1 offenbart ein Epilationsgerät, das eine Spannungsquelle und wenigstens eine Elektrode aufweist, so daß insbesondere vor dem Auszupfen der Haare auf der Haut ein elektrischer Funken erzeugbar ist. Durch die Funkenentladung wird die Nervenstimulation derart positiv beeinflußt, daß eine Reduzierung des Schmerzempfindens beim Auszupfen der Haare erreicht wird.

Der Erfindung liegt die Aufgabe zugrunde, beim Auszupfen von Haaren aus der Haut mittels eines Epilationsgeräts beim Benutzer ein möglichst geringes Schmerzempfinden hervorzurufen.

Diese Aufgabe wird durch die Merkmalskombination des Anspruchs 1 gelöst.

Das erfindungsgemäße Epilationsgerät, insbesondere zum Auszupfen von Haaren der menschlichen Haut, weist ein in der Hand haltbares Gehäuse, eine motorisch angetriebene Klemmvorrichtung zum Erfassen und Auszupfen der Haare, eine Einrichtung zur Erzeugung eines Stromsignals und wenigstens zwei Elektroden zum Einleiten des Stromsignals in die Haut auf. Die Besonderheit des erfindungsgemäßen Epilationsgeräts besteht darin, daß das Stromsignal eine Frequenz im Kilohertzbereich und eine Effektivstromstärke im Bereich zwischen 0,1 mA und 15 mA aufweist.

Die Erfindung hat den Vorteil, daß das Schmerzempfinden beim Auszupfen der Haare gegenüber einem herkömmlichen Epilationsgerät erheblich reduziert ist. Durch das erfindungsgemäß ausgebildete Stromsignal läßt sich zudem auch gegenüber Epilationsgeräten, bei denen bereits eine Stromanwendung vorgesehen ist, eine nochmalige Reduzierung des Schmerzempfindens erreichen. Dadurch kann die Akzeptanz von Epilationsgeräten bei potentiellen Benutzern erhöht werden.

Besonders gute Ergebnisse hinsichtlich der Reduzierung des Schmerzempfindens lassen sich erzielen, wenn das Stromsignal eine Frequenz zwischen 3 kHz und 20 kHz, vorzugsweise etwa 10 kHz aufweist. Günstige Werte für die Effektivstromstärke des Stromsignals liegen im Bereich zwischen 0,5 mA und 10 mA, vorzugsweise zwischen 0,7 mA und 1,3 mA.

Bei einem bevorzugten Ausführungsbeispiel des erfindungsgemäßen Epilationsgeräts ist das Stromsignal moduliert, vorzugsweise amplitudenmoduliert. Die Modulationsfrequenz kann zwischen 40 Hz und 120 Hz betragen. Besonders günstig ist ein Wert von etwa 100 Hz. Die Modulationstiefe des Stromsignals kann zwischen 50 % und 100 % betragen. Weiterhin kann das Stromsignal sinusförmig ausgebildet und/oder sinusförmig moduliert sein.

Für eine Erzielung einer möglichst starken Reduzierung des Schmerzempfindens spielt der zeitliche Zusammenhang zwischen der Einwirkung des Stromsignals und dem schmerzauslösenden Ereignis eine wichtige Rolle. In dieser Hinsicht ist es von Vorteil, wenn das Stromsignal 480 ms bis 0 ms vor Beginn eines tatsächlichen oder potentiellen Zupfereignisses in die Haut einleitbar ist. Weiterhin ist es vorteilhaft, wenn das Stromsignal im Bereich von 80 ms vor bis 80 ms nach Beginn des tatsächlichen oder potentiellen Zupfereignisses endet. Als Dauer für das Stromsignal eignet sich insbesondere ein Wert von etwa 10 ms.

Um beispielsweise das Einleiten des Stromsignal entsprechend dem vorstehend beschriebenen Zeitrahmen auf das Zupfereignis abzustimmen, kann die Einrichtung zur Erzeugung des Stromsignals mit einem Geber zur Erzeugung eines Triggersignals gekoppelt sein. Insbesondere kann das Triggersignal mit dem tatsächlichen oder potentiellen Zupfereignis korreliert sein. Weiterhin kann die Einrichtung zur Erzeugung des Stromsignals einen Funktionsgenerator zur Erzeugung einer gewünschten Signalform für das Stromsignal aufweisen.

Um das Stromsignal zu begrenzen, kann den Elektroden wenigstens ein Widerstand vorgeschaltet sein, dessen Widerstandswert größer ist als eine durchschnittliche Impedanz der Haut und vorzugsweise etwa 100 k? beträgt. Ein großer Widerstand hat den Vorteil, daß sich Schwankungen der Impedanz der Haut nicht so stark auf das Stromsignal auswirken als dies bei einem kleinen Widerstand der Fall ist. Dadurch kann auf Maßnahmen zur Reduzierung der Impedanz der Haut verzichtet werden. Derartige Maßnahmen zielen in der Regel auf eine Anfeuchtung der Haut ab und wirken sich zumeist negativ auf das Epilationsergebnis aus.

Die Elektroden sind vorzugsweise an einem abnehmbaren Elektrodenträger angeordnet. Dies erleichtert die Reinigung des Epilationsgeräts. Außerdem besteht die Möglichkeit das Epilationsgerät in herkömmlicher Weise ohne Stromeinwirkung zu betreiben. Besonders zweckmäßig ist es, wenn die Elektroden als Metallrollen ausgebildet sind.

Um den Benutzer eine Anpassung an seine Bedürfnisse zu ermöglichen, ist es von Vorteil, wenn das erfindungsgemäße Epilationsgerät ein Einstellelement zur Beeinflussung des Stromsignals aufweist.

Die Erfindung bezieht sich weiterhin auf ein Verfahren zum Auszupfen von Haaren der menschlichen Haut mittels eines Epilationsgeräts, das über die Haut geführt wird und dabei die Haare erfaßt und aus der Haut auszupft. Dabei wird vor oder während des Auszupfens der Haare ein Stromsignal in die Haut eingeleitet. Das erfindungsgemäße Verfahren zeichnet sich dadurch aus, daß das Stromsignal eine Frequenz im Kilohertzbereich und eine Effektivstromstärke im Bereich zwischen 0,1 mA und 15 mA aufweist.

Gemäß einer ersten Variante des erfindungsgemäßen Verfahrens wird das Stromsignal während des Epilationsvorgangs mit Hilfe des Epilationsgeräts in die Haut eingeleitet. Dies hat den Vorteil, daß der Ablauf eines konventionellen Epilationsvorgangs unverändert beibehalten werden kann.

Bei einer zweiten Variante des erfindungsgemäßen Verfahrens wird mit der Stromeinleitung in die Haut bereits vor Beginn des Epilationsvorgangs begonnen. Das Stromsignal kann bei der zweiten Variante 1 Minute bis 10 Minuten, vorzugsweise 3 Minuten bis 6 Minuten lang in die Haut eingeleitet werden. Besonders vorteilhaft ist es, wenn die Stromeinleitung in die Haut vor Beginn, insbesondere unmittelbar vor Beginn, des Epilationsvorgangs beendet wird. Dadurch besteht die Möglichkeit, die Epilation nach einer derartigen Vorbehandlung mit einem konventionellen Epilationsgerät bei einem reduzierten Schmerzempfinden auszuführen.

Die Erfindung wird nachstehend anhand des in der Zeichnung dargestellten Ausführungsbeispiels näher erläutert.

Es zeigen:
- Fig. 1: ein Ausführungsbeispiel eines Epilationsgeräts gemäß der Erfindung in perspektivischer Darstellung und
- Fig. 2: eine schematische Darstellung für die erfindungsgemäße Vorgehensweise bei der Einleitung der Stromsignale in die Haut.

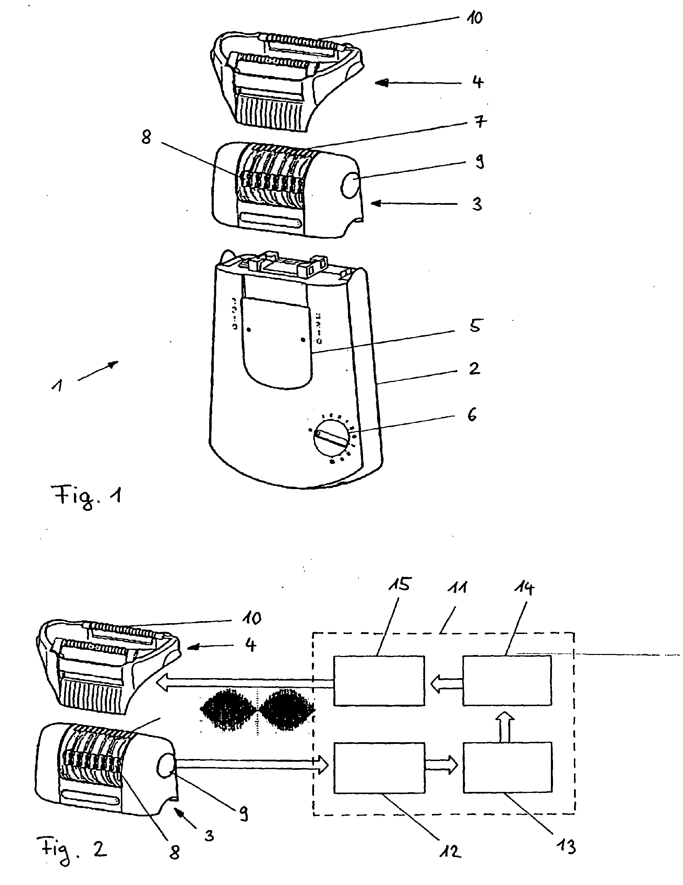

Fig. 1 zeigt ein Ausführungsbeispiel eines Epilationsgeräts 1 gemäß der Erfindung in perspektivischer Darstellung. Das Epilationsgerät 1 weist ein Gehäuse 2, einen abnehmbar am Gehäuse 2 befestigten Epilationskopf 3 und einen abnehmbar am Epilationskopf 3 befestigten Elektrodenträger 4 auf. Am Gehäuse 2 sind ein Schalter 5 und ein Einstellknopf 6 angeordnet. Der Epilationskopf 3 weist eine drehbar gelagerte Epilationswalze 7 mit drei gleichmäßig über den Umfang verteilten Reihen von Pinzetten 8 auf. Weiterhin weist der Epilationskopf 3 einen Drehwinkelgeber 9 zur Erfassung der Drehwinkelposition der Epilationswalze 7 auf. Die Epilationswalze 7 kann von einem nicht figürlich dargestellten Elektromotor in Rotation versetzt werden, der innerhalb des Gehäuses 2 angeordnet ist. Die Drehzahl der Epilationswalze 7 beträgt typischerweise etwa 750 Umdrehungen pro Minute. Beim Rotieren der Epilationswalze 7 öffnen und schließen sich die Pinzetten 8 und können dabei Haare erfassen und auszupfen. Der Drehwinkelgeber 9 kann beispielsweise so ausgebildet sein, daß er immer dann, wenn die Pinzetten 8 im Bereich des vom Gehäuse 2 abgewandten Endes des Epilationskopfes 3 gerade geschlossen sind, ein Triggersignal ausgibt. Eine derartige Funktion des Drehwinkelgebers 9 läßt sich mittels einer Kontaktanordnung an einem Widerlager für ein Element zur Betätigung der Pinzetten 8 realisieren.

Der Elektrodenträger 4 weist zwei parallel zueinander angeordnete Elektroden 10 auf, die beim dargestellten Ausführungsbeispiel als Metallrollen ausgebildet sind. Der Durchmesser dieser Metallrollen beträgt typischerweise jeweils etwa 4 mm. Die Oberfläche jeder Elektrode 10 beträgt jeweils etwa 35 mm². Als Material für die Elektroden 10 kann beispielsweise Messing eingesetzt werden. Wenn der Elektrodenträger 4 auf den Epilationskopf 3 aufgesetzt ist, sind die Elektroden 10 beidseits neben der Epilationswalze 7 angeordnet und achsparallel zur Epilationswalze 7 ausgerichtet. Die Elektroden 10 sind mittels nicht figürlich dargestellter Federn so vorgespannt, daß sie federnd über die freien Enden der Pinzetten 8 überstehen, wenn die Pinzetten 8 mittig zwischen den Elektroden 10 angeordnet sind. Das Epilationsgerät 1 kann auch so abgewandelt werden, daß der Elektrodenträger 4 nicht erforderlich ist. Hierzu können die Elektroden 10 auf der Epilationswalze 7 angeordnet werden oder durch die Pinzetten 8 selbst gebildet werden.

Zur Durchführung des Epilationsvorgangs wird am Einstellknopf 6 eine im folgenden noch näher erläuterte Einstellung vorgenommen und das Epilationsgerät 1 mittels des Schalters 5 in Betrieb genommen. Dann wird der Epilationskopf 3 von einem Benutzer über einen Hautbereich geführt, in dem die in der Haut verankerten Haare ausgezupft werden sollen. Die Haare werden von den mit der Epilationswalze 7 rotierenden Pinzetten 8 erfaßt und aus der Haut ausgezupft. Das Auszupfen der Haare ist für den Benutzer jeweils mit einem Schmerz verbunden. Um diesen Schmerz für den Benutzer möglichst gering zu halten, werden im Rahmen der Erfindung Stromsignale in die Haut eingeleitet, durch die das Schmerzempfinden des Benutzers reduziert wird. Durch die Stromsignale werden zusätzlich zu den Nervenfasern, die den Schmerzreiz weiterleiten, weitere Nervenfasern aktiviert. Dies führt bedingt durch Mechanismen der Signalverarbeitung im zentralen Nervensystem zu einer Reduzierung des Schmerzempfindens. Die Einleitung der Stromsignale in die Haut wird im einzelnen anhand von Fig. 2 erläutert.

Fig. 2 zeigt eine schematische Darstellung für die erfindungsgemäße Vorgehensweise bei der Einleitung der Stromsignale in die Haut. Die Stromsignale werden jeweils von einer Einrichtung 11 erzeugt, deren Komponenten und Funktionsweise im folgenden näher beschrieben werden und mit Hilfe der Elektroden 10 in die Haut eingeleitet. Für die Erzeugung eines Stromsignals wird das vom Drehwinkelgeber 9 ausgegebene Triggersignal in einen Funktionsgenerator 12 eingespeist. Der Funktionsgenerator 12 erzeugt in einer festen Zeitkorrelation zum Triggersignal ein periodisches Spannungssignal, beispielsweise mit einer Sinus-, Rechteck-, Dreieck- oder Sägezahnform. Die Sinusform wird dabei bevorzugt eingesetzt. Das Spannungssignal weist eine Frequenz im Kilohertzbereich auf. Insbesondere liegt die Frequenz zwischen 3 und 20 kHz. Im vorliegenden Ausführungsbeispiel beträgt die Frequenz 10 kHz. Das Spannungssignal ist moduliert, wobei beispielsweise eine Amplitudenmodulation mit Sinus-, Rechteck-, Dreieck- oder Sägezahnform gewählt werden kann. Die Modulationsfrequenz beträgt zwischen 40 Hz und 120 Hz, die Modulationstiefe 50 bis 100 %. Die Modulation erfolgt vorzugsweise bipolar und nulliniensymmetrisch. Im vorliegenden Ausführungsbeispiel wird ein sinusförmig amplitudenmoduliertes Spannungssignal mit einer Modulationsfrequenz von 100 Hz und einer Modulationstiefe von 100 % eingesetzt, wobei die Modulation bipolar und nulliniensymmetrisch ausgebildet ist.

Das vom Funktionsgenerator 12 erzeugte Spannungssignal wird in einen Verstärker 13 eingespeist, dessen Verstärkungsfaktor mit Hilfe des Einstellknopfes 6 einstellbar ist. Über einen Übertrager 14 und wenigstens einen Widerstand 15 wird das vom Verstärker 13 ausgegebene Spannungssignal an die Elektroden 10 des Epilationsgeräts 1 angelegt. Je nach dem, welcher Verstärkungsfaktor mit dem Einstellknopf 6 eingestellt wurde, beträgt der Spitze-Spitze-Wert der an den Elektroden 10 anliegenden Leerlaufspannung bis ca. 2,5 kV. Die Einstellung kann vom Benutzer empirisch so vorgenommen werden, daß sich eine möglichst ausgeprägte schmerzreduzierende Wirkung ergibt. Die Leerlaufspannung liegt jeweils dann an, wenn die Elektroden 10 keinen Hautkontakt haben.

Der Widerstand 15 begrenzt das über die Elektroden 10 in die Haut eingeleitete Stromsignal und ist vorzugsweise groß gegenüber der durchschnittlichen Impedanz der Haut. Dadurch ist gewährleistet, daß bei unterschiedlichen Werten für die Hautimpedanz, die beispielsweise bei verschiedenen Benutzern des Epilationsgeräts 1 oder beim gleichen Benutzer zu verschiedenen Zeiten auftreten können, das in die Haut eingeleitete Stromsignal nicht allzu stark variiert. Auf das Auftragen von Kontaktgelen auf die Haut oder die Verwendung von feuchtigkeitsspendenden Elektroden 10 kann daher verzichtet werden. Das Stromsignal sollte eine Effektivstromstärke im Bereich zwischen 0,1 und 15 mA, vorzugsweise zwischen 0,5 und 10 mA aufweisen. Im dargestellten Ausführungsbeispiel wird ein Stromsignal mit einer Effektivstromstärke zwischen 0,7 und 1,3 mA in die Haut eingeleitet. Der Widerstand 15 weist einen Wert von etwa 100 k? auf.

Für eine möglichst ausgeprägte Reduzierung des Schmerzempfindens während des Epilationsvorgangs ist es wichtig eine räumliche und insbesondere auch eine zeitliche Korrelation zwischen dem schmerzauslösenden Ereignis und dem Einleiten des Stromsignals in die Haut einzuhalten. Das schmerzauslösende Ereignis stellt jeweils das Auszupfen eines Haares dar, wobei der Schmerz unmittelbar nach dem Erfassen des Haares mit der Pinzette 8 beginnt, wenn sich die Pinzette 8 mit dem Haar weiterbewegt und dadurch am Haar zieht. Die räumliche Korrelation ist durch die Anordnung der Elektroden 10 beidseits neben der Epilationswalze 7 gegeben. Dadurch ist gewährleistet, daß eine Stromeinwirkung jeweils am Auszupfort und in dessen Umgebung stattfindet. Die zeitliche Korrelation kann jeweils mit Hilfe des vom Drehwinkelgeber 9 erzeugten Triggersignals hergestellt werden. Mit dem Triggersignal lassen sich unter anderem jeweils die Zeitpunkte ermitteln, zu denen sich die Pinzetten 8 in der Nähe der Haut schließen und somit potentiell ein oder mehrere Haare erfaßt werden. Mit anderem Worten, dem Funktionsgenerator 12 ist jeweils der Beginn potentieller Zupfereignisse bekannt. Korreliert dazu erzeugt der Funktionsgenerator 12 seine Spannungssignale so, daß mit der Einleitung der dadurch hervorgerufenen Stromsignale in die Haut jeweils in einem Zeitintervall von 480 ms bis 0 ms vor dem Beginn des Zupfereignisses begonnen wird. Die Stromeinleitung endet im einem Zeitintervall 80 ms vor bis 80 ms nach Beginn des Zupfereignisses. Beim vorliegenden Ausführungsbeispiel beginnt das Stromsignal mit dem Beginn des Zupfereignisses und weist eine Länge von 10 ms auf. Dies entspricht bei der gewählten Modulationsfrequenz von 100 Hz gerade einer Modulationsperiode, wobei das Stromsignal mit einem Nulldurchgang der modulierenden Sinusfunktion beginnt und mit einem weiteren Nulldurchgang endet.

Bei einer Weiterbildung der erfindungsgemäßen Vorgehensweise ist es vorgesehen, nur dann ein Stromsignal in die Haut einzuleiten, wenn tatsächlich wenigstens ein Haar von den Pinzetten 8 erfaßt wurde. Dadurch soll vermieden werden, daß eine Stromeinleitung ohne Zupfereignis und damit ohne schmerzauslösendes Ereignis erfolgt. Bei dieser Weiterbildung weist das Epilationsgerät 1 einen Detektor auf, der beispielsweise auf elektrischem oder mechanischem Weg feststellt, ob von den Pinzetten 8 wenigstens ein Haar erfaßt wurde.

Alternativ zur geschilderten Vorgehensweise, bei der die Stromeinleitung im Rahmen des Epilationsvorgangs erfolgt, ist es auch möglich, die Einleitung der Stromsignale in die Haut und den Epilationsvorgang getrennt voneinander durchzuführen. Bei dieser Variante werden die Stromsignale vor dem Epilationsvorgang über mindestens zwei Elektroden 10, die nicht am Epilationsgerät 1 angebracht sein müssen, in die Haut eingeleitet. Dabei müssen sich die Elektroden 10 auch nicht in unmittelbarer Nähe zum vorgesehenen Epilationsgebiet befinden. Die Elektroden 10 müssen lediglich im gleichen nervalen Versorgungsgebiet, in dem auch der Epilationsvorgang vorgesehen ist oder über dem dieses Gebiet versorgenden Nerv angebracht werden. Unter Berücksichtigung der jeweiligen Neuronanatomie können Flächen von etwa 20 cm² bis 150 cm² bei einem Elektrodenabstand von 5 cm bis 15 cm behandelt werden. Dabei wird das Stromsignal permanent über einen Zeitraum von etwa 3 bis 6 Minuten appliziert. Danach wird mit dem Epilationsvorgang begonnen. Da die schmerzreduzierende Wirkung nach dem Abschalten des Stromsignals noch mindestens 5 Minuten lang anhält, kann die Stromanwendung wahlweise beibehalten oder vor Beginn des Epilationsvorgangs beendet werden. Die Effektivstromstärke wird bei der dem Epilationsvorgang vorgelagerten Stromanwendung geringfügig niedriger gewählt als bei der eingangs beschriebenen Stromanwendung während des Epilationsvorgangs. Abweichend von der eingangs beschriebenen Variante können nunmehr auch kurze, nulliniensymmetrische Rechteckpulse in einem Frequenzbereich von 100 bis 200 Hz verwendet werden.

## Patentansprüche

1. Epilationsgerät, mit einem in der Hand haltbaren Gehäuse (2), einer motorisch angetriebenen Klemmvorrichtung (7, 8) zum Erfassen und Auszupfen der Haare, einer Einrichtung (11) zur Erzeugung eines Stromsignals und wenigstens zwei am Epilationsgerät angebrachten Elektroden (10) zum Einleiten des Stromsignals in die Haut, **dadurch gekennzeichnet, dass** das Stromsignal eine Frequenz zwischen 3 kHz und 20 kHz, vorzugsweise etwa 10 kHz, und eine Effektivstromstärke im Bereich zwischen 0,1 mA und 15 mA aufweist.

2. Epilationsgerät nach einem der vorhergehenden Ansprüche, **dadurch gekennzeichnet, dass** das Stromsignal eine Effektivstromstärke im Bereich zwischen 0,5 mA und 10 mA, vorzugsweise zwischen 0,7 mA und 1,3 mA aufweist.

3. Epilationsgerät nach einem der vorhergehenden Ansprüche, **dadurch gekennzeichnet, dass** das Stromsignal moduliert, vorzugsweise amplitudenmoduliert ist.

4. Epilationsgerät nach Anspruch 3, **dadurch gekennzeichnet, dass** das Stromsignal mit einer Modulationsfrequenz von 40 Hz bis 120 Hz, vorzugsweise etwa 100 Hz moduliert ist.

5. Epilationsgerät nach einem der Ansprüche 3 oder 4, **dadurch gekennzeichnet, dass** das Stromsignal mit einer Modulationstiefe von 50 % bis 100 % moduliert ist.

6. Epilationsgerät nach einem der vorhergehenden Ansprüche, **dadurch gekennzeichnet, dass** das Stromsignal sinusförmig ausgebildet und/oder sinusförmig moduliert ist.

7. Epilationsgerät nach einem der vorhergehenden Ansprüche, **dadurch gekennzeichnet, dass** das Stromsignal 480 ms bis 0 ms vor Beginn eines tatsächlichen oder potentiellen Zupfereignisses in die Haut einleitbar ist.

8. Epilationsgerät nach einem der vorhergehenden Ansprüche, **dadurch gekennzeichnet, dass** das Stromsignal im Bereich von 80 ms vor bis 80 ms nach Beginn des tatsächlichen oder potentiellen Zupfereignisses endet.

9. Epilationsgerät nach einem der vorhergehenden Ansprüche, **dadurch gekennzeichnet, dass** die Dauer des Stromsignals etwa 10 ms beträgt.

10. Epilationsgerät nach einem der vorhergehenden Ansprüche, **dadurch gekennzeichnet, dass** die Einrichtung (11) zur Erzeugung des Stromsignals mit einem Geber (9) zur Erzeugung eines Triggersignals gekoppelt ist.

11. Epilationsgerät nach Anspruch 10, **dadurch gekennzeichnet, dass** das Triggersignal mit dem tatsächlichen oder potentiellen Zupfereignis korreliert ist.

12. Epilationsgerät nach einem der vorhergehenden Ansprüche, **dadurch gekennzeichnet, dass** die Einrichtung (11) zur Erzeugung des Stromsignals einen Funktionsgenerator (12) zur Erzeugung einer gewünschten Signalform für das Stromsignal aufweist.

13. Epilationsgerät nach einem der vorhergehenden Ansprüche, **dadurch gekennzeichnet, dass** den Elektroden (10) wenigstens ein Widerstand (15) vorgeschaltet ist, dessen Widerstandswert größer ist als eine durchschnittliche Impedanz der Haut und vorzugsweise etwa 100 kΩ beträgt.

14. Epilationsgerät nach einem der vorhergehenden Ansprüche, **dadurch gekennzeichnet, dass** die Elektroden (10) an einem abnehmbaren Elektrodenträger (4) angeordnet sind.

15. Epilationsgerät nach einem der vorhergehenden Ansprüche, **dadurch gekennzeichnet, dass** die Elektroden (10) als Metallrollen ausgebildet sind.

16. Epilationsgerät nach einem der vorhergehenden Ansprüche, **dadurch gekennzeichnet, dass** es ein Einstellelement (6) zur Beeinflussung des Stromsignals aufweist.

17. Verfahren zum Auszupfen von Haaren der menschlichen Haut mittels eines Epilationsgeräts (1), das über die Haut geführt wird und dabei die Haare erfasst und aus der Haut auszupft, wobei vor oder während des Auszupfens der Haare ein Stromsignal über Elektroden, die am Epilationsgerät (1) angeordnet sind, in die Haut eingeleitet wird, **dadurch gekennzeichnet, dass** das Stromsignal eine Frequenz zwischen 3 kHz und 20 kHz, vorzugsweise etwa 10 kHz, und eine Effektivstromstärke im Bereich zwischen 0,1 mA und 15 mA aufweist.

18. Verfahren nach Anspruch 17, **dadurch gekennzeichnet, dass** das Stromsignal während des Epilationsvorgangs mit Hilfe des Epilationsgeräts (1) in die Haut eingeleitet wird.

## Claims

1. Epilator having a housing (2) which can be held in the hand, a motor-driven pinching apparatus (7, 8) for grasping and plucking the hairs, a device (11) for generating a current signal, and at least two electrodes (10), which are mounted on the epilator, for discharging the current signal into the skin, **characterized in that** the current signal has a frequency of between 3 kHz and 20 kHz, preferably approximately 10 kHz, and an effective current intensity in the range of between 0.1 mA and 15 mA.

2. Epilator according to Claim 1, **characterized in that** the current signal has an effective current intensity in the range of between 0.5 mA and 10 mA, preferably between 0.7 mA and 1.3 mA.

3. Epilator according to either of the preceding claims, **characterized in that** the current signal is modulated, preferably amplitude-modulated.

4. Epilator according to Claim 3, **characterized in that** the current signal is modulated at a modulation frequency of from 40 Hz to 120 Hz, preferably approximately 100 Hz.

5. Epilator according to either of Claims 3 and 4, **characterized in that** the current signal is modulated at a modulation level of from 50% to 100%.

6. Epilator according to one of the preceding claims, **characterized in that** the current signal is sinusoidal and/or sinusoidally modulated.

7. Epilator according to one of the preceding claims, **characterized in that** the current signal can be discharged into the skin 480 ms to 0 ms before an actual or potential plucking event begins.

8. Epilator according to one of the preceding claims, **characterized in that** the current signal ends in the range of from 80 ms before to 80 ms after the beginning of the actual or potential plucking event.

9. Epilator according to one of the preceding claims, **characterized in that** the duration of the current signal is approximately 10 ms.

10. Epilator according to one of the preceding claims, **characterized in that** the device (11) for generating the current signal is coupled to a transmitter (9) for generating a trigger signal.

11. Epilator according to Claim 10, **characterized in that** the trigger signal is correlated with the actual or potential plucking event.

12. Epilator according to one of the preceding claims, **characterized in that** the device (11) for generating the current signal has a function generator (12) for generating a desired signal form for the current signal.

13. Epilator according to one of the preceding claims, **characterized in that** at least one resistor (15) is connected upstream of the electrodes (10), the resistance value of the said resistor being greater than an average impedance of the skin and preferably being approximately 100 kΩ.

14. Epilator according to one of the preceding claims, **characterized in that** the electrodes (10) are arranged on a removable electrode carrier (4).

15. Epilator according to one of the preceding claims, **characterized in that** the electrodes (10) are in the form of metal rollers.

16. Epilator according to one of the preceding claims, **characterized in that** it has an adjusting element (6) for influencing the current signal.

17. Method for plucking hairs from human skin by means of an epilator (1) which is passed over the skin and in the process grasps the hairs and plucks the said hairs from the skin, with a current signal being discharged into the skin by means of electrodes which are arranged on the epilator (1) before or during the process of plucking the hairs, **characterized in that** the current signal has a frequency of between 3 kHz and 20 kHz, preferably approximately 10 kHz, and an effective current intensity in the range of between 0.1 mA and 15 mA.

18. Method according to Claim 17, **characterized in that** the current signal is discharged into the skin with the aid of the epilator (1) during the epilation process.

## Revendications

1. Appareil épilatoire comprenant un boîtier (2) pouvant être tenu à la main, un dispositif de serrage (7, 8) à entraînement motorisé pour saisir et arracher les poils, un dispositif (11) pour générer un signal électrique et au moins deux électrodes (10) montées sur l'appareil épilatoire pour injecter le signal électrique dans la peau, **caractérisé en ce que** le signal électrique présente une fréquence comprise entre 3 kHz et 20 kHz, de préférence égale à environ 10 kHz, et une intensité de courant efficace dans la plage de 0,1 mA et 15 mA.

2. Appareil épilatoire selon la revendication 1, **caractérisé en ce que** le signal électrique présente une intensité de courant efficace comprise dans la plage entre 0,5 mA et 10 mA, de préférence entre 0,7 mA et 1,3 mA.

3. Appareil épilatoire selon l'une des revendications précédentes, **caractérisé en ce que** le signal électrique est modulé, de préférence modulé en amplitude.

4. Appareil épilatoire selon la revendication 3, **caractérisé en ce que** le signal électrique est modulé avec une fréquence de modulation de 40 Hz à 120 Hz, de préférence d'environ 100 Hz.

5. Appareil épilatoire selon l'une des revendications 3 ou 4, **caractérisé en ce que** le signal électrique est modulé avec une profondeur de modulation de 50 % à 100 %.

6. Appareil épilatoire selon l'une des revendications précédentes, **caractérisé en ce que** le signal électrique est de forme sinusoïdale et/ou modulé de manière sinusoïdale.

7. Appareil épilatoire selon l'une des revendications précédentes, **caractérisé en ce que** le signal électrique peut être injecté dans la peau 480 ms à 0 ms avant le début d'une action d'arrachage réelle ou potentielle.

8. Appareil épilatoire selon l'une des revendications précédentes, **caractérisé en ce que** le signal électrique se termine dans la plage de 80 ms avant à 80 ms après le début d'une action d'arrachage réelle ou potentielle.

9. Appareil épilatoire selon l'une des revendications précédentes, **caractérisé en ce que** la durée du signal électrique est de 10 ms.

10. Appareil épilatoire selon l'une des revendications précédentes, **caractérisé en ce que** le dispositif (11) de génération du signal électrique est connecté à un codeur (9) pour produire un signal de déclenchement.

11. Appareil épilatoire selon la revendication 10, **caractérisé en ce que** le signal de déclenchement est corrélé avec l'action d'arrachage réelle ou potentielle.

12. Appareil épilatoire selon l'une des revendications précédentes, **caractérisé en ce que** le dispositif (11) de génération du signal électrique présente un générateur de fonction (12) pour générer une forme de signal souhaitée pour le signal électrique.

13. Appareil épilatoire selon l'une des revendications précédentes, **caractérisé en ce qu'**au moins une résistance (15) est branchée en amont des électrodes (10), dont la valeur est supérieure à une impédance moyenne de la peau et de préférence égale à environ 100 kΩ.

14. Appareil épilatoire selon l'une des revendications précédentes, **caractérisé en ce que** les électrodes (10) sont disposées sur un porte-électrode (4) amovible.

15. Appareil épilatoire selon l'une des revendications précédentes, **caractérisé en ce que** les électrodes (10) sont réalisées sous la forme de rouleaux métalliques.

16. Appareil épilatoire selon l'une des revendications précédentes, **caractérisé en ce qu'**il présente un élément de réglage (6) destiné à influencer le signal électrique.

17. Procédé d'extraction de poils de la peau humaine au moyen d'un appareil épilatoire (1) qui est passé sur la peau et qui saisit alors les poils et les arrache de la peau, un signal électrique étant injecté dans la peau avant ou pendant l'arrachage des poils par le biais d'électrodes qui sont disposées sur l'appareil épilatoire (1), **caractérisé en ce que** le signal électrique présente une fréquence comprise entre 3 kHz et 20 kHz, de préférence égale à environ 10 kHz, et une intensité de courant efficace dans la plage de 0,1 mA et 15 mA.

18. Procédé selon la revendication 17, **caractérisé en ce que** le signal électrique est injecté dans la peau à l'aide de l'appareil épilatoire (1) pendant l'opération d'épilation.
